# EUROPEAN PATENT SPECIFICATION

(11) **EP 2 329 784 B1**
(45) Date of publication and mention of the grant of the patent: **10.12.2014**
(21) Application number: 10188313.0
(22) Date of filing: 21.10.2010
(51) Int. Cl.: A61B 18/14

(54) **Improved RF Appartus for treating slight blemishes and/or pathologies of a human body**
Verbesserte HF Apparatur zur Behandlung von leichten Hautmakeln und/oder Pathologien eines menschlichen Körpers
Appareils RF amélioré pour le traitement des imperfections mineures de la peau et / ou la pathologie du corps humain

(30) Priority: 04.12.2009 IT MI20092153
(43) Date of publication of application: 08.06.2011
(73) Proprietor: Novavision Group S.p.A., 20123 Milano (IT)
(72) Inventor: Crapelli, Danilo, 20123, Milano (IT); Battista, Riccardo, 00124, Roma (IT)
(74) Representative: Cicogna, Franco

(56) References cited:
- WO-A2-2007/088541
- WO-A2-2008/072237

## Description

The present invention relates to an improved RF apparatus for treating slight blemishes and/or pathologies of a human body.

As is known, a radiofrequency radiation may be considered as a sub-set of electromagnetic radiation having wavelengths compressed from 100 Km and 1 mm, respectively at frequencies from 300 Hz to 3000 GHz.

This electromagnetic radiation range constitutes the radio spectrum and corresponds to the frequency of A.C. electric signals used for generating and/or detecting radiowaves.

The above mentioned radiofrequencies may relate to the electromagnetic oscillations in electric circuits or air and space radiations.

Likewise to other electromagnetic radiation sub-sets, radiofrequency waves are propagated at the light speed.

The application of a radiofrequency for treating or processing human skin is based on a well consolidated physical principle, according to which the energy or power supplied by a RF processing or treating instrument or tool is transformed into heat upon contacting a human skin.

This mechanism is due to the natural resistance (impedance) of the cutaneous tissues to movable electrons driven by a radiofrequency field and is proportional to the supplied current amount and the provided application time.

The above impedance increases at cutaneous or skin regions whereat the derm layer and undercutaneous fat have a larger thickness, for example on the patient trunk, thighs and arms.

A RF apparatus, based on a 1 MHz RF generator with a four-pole applicator controlled by a microprocessor allowing to select which poles must operate as "a transmitter" and which must operate as "a receiver" is already known.

The above four-pole resistive system allows to focalize power at a target point to provide desired treatment results by simultaneous heating both the derm and hypoderm layer surfaces.

Thus, electric current would be concentrated and limited to the inside of the four electrodes, thereby providing a high RF density assuring good treatment results in early treatment or processing operations, while using an energy low contents.

A skin processing by using the above mentioned RF apparatus allows to process human body regions having cutaneous slackened areas, such as the human face, nose-lip grooves, cheek and peri-orbital areas, front and neck, or abdomen and thigh inner regions, gluteus, under-gluteal regions and arm inner regions.

The objective is that to increase the fat cell and collagen fiber temperature, to provide a controlled inflammation thereof.

Thus, a further principle thereon the RF processing or treatment device is based, is heat transmission, which has been found to be able of generating a concentration of the existing collagen and a new collagen formation.

However, the use of a RF treatment on a human body, and in particular on the human skin, may cause the skin at the processed region to redden and a formation of slight edemas.

A device according to the preamble of claim 1 is known from WO 2007 088 541.

### SUMMARY OF THE INVENTION

Accordingly, the aim of the present invention is to provide such an improved RF apparatus for treating slight blemishes and/or pathologies of a human body, overcoming the mentioned drawbacks of the prior art.

Within the scope of the above mentioned aim, a main object of the invention is to provide such an improved RF apparatus reducing to a minimum possible collateral and undesired RF effects, as a RF radiation is applied to a human body for treating slight blemishes and/or pathologies.

Another object of the present invention is to provide such an improved RF frequency apparatus able of aiding an operator to perform an efficient and safe skin treatment.

Yet another object of the present invention is to provide such an improved RF apparatus which, owing to its specifically designed constructional features, is very reliable and safe in operation.

According to one aspect of the present invention which is defined in claim 1, the above mentioned aim and objects, as well as yet other objects, which will become more apparent hereinafter, are achieved by an improved RF apparatus for treating slight blemishes and/or pathologies of a human body, characterized in that said apparatus comprises an electronically controlled RF emitter, a RF source, a control device and a man-machine interface.

Said emitter comprises a plurality of electric contacts made of a biocompatible material, said electric contacts coupling said RF source to said human body, a movement sensor to detect a displacement of said emitter, and a temperature sensor to detect a temperature increase of a portion of said human body being treated.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example, in the accompanying drawings, where:

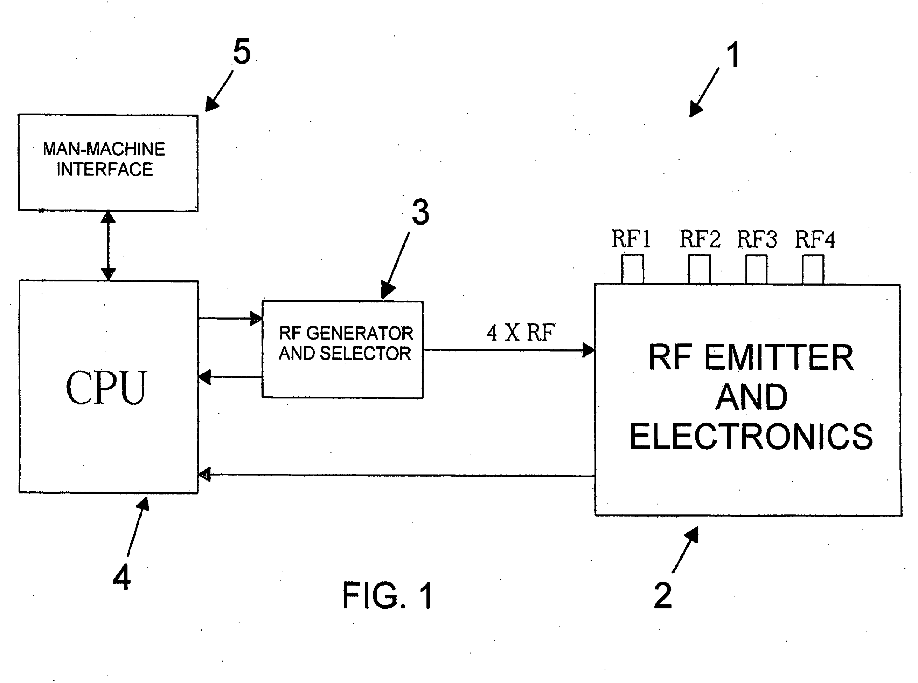

Figure 1 shows a block diagram of the improved RF apparatus for treating slight blemishes and/or pathologies of a human body according to the present invention;

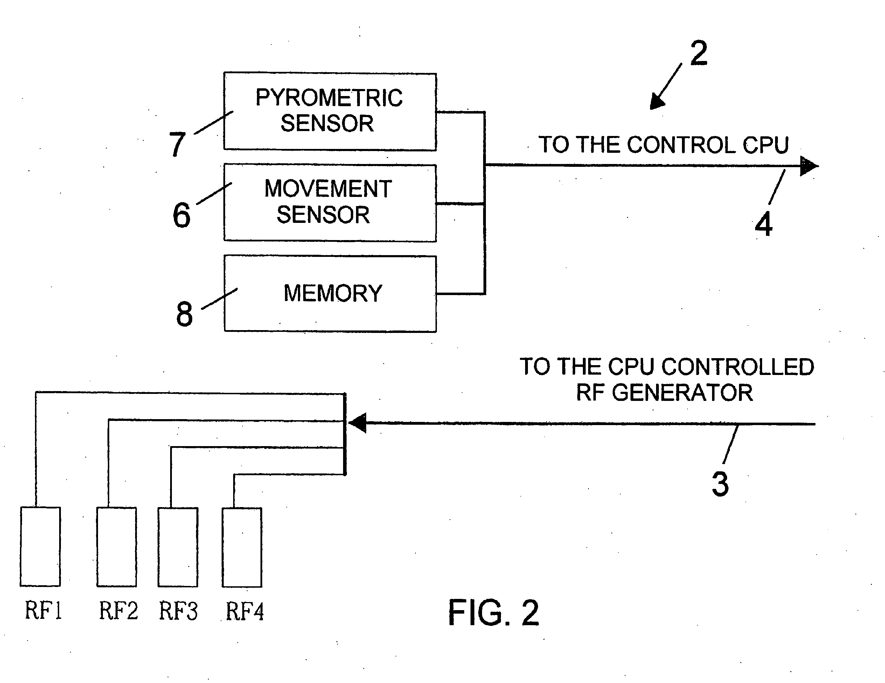

Figure 2 shows a block diagram of the emitter included in the improved RF apparatus for treating slight blemishes and/or pathologies of a human body;

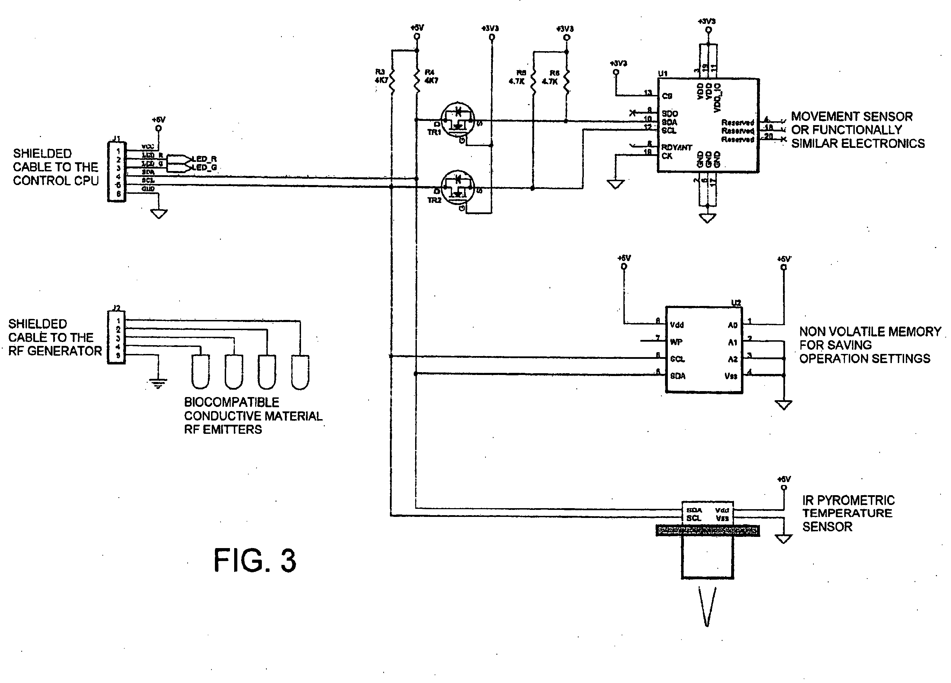

Figure 3 shows and electric diagram of the emitter shown in figure 2;
   and
Figure 4 shows a diagram of the apparatus handle constituting an integrating part of the inventive apparatus.

### DESCRIPTION OF THE PREFERRED EMBODIMENTS

With reference to the number references of the above mentioned figures, the improved RF apparatus for treating slight blemishes and/or pathologies of a human body, according to the present invention, which has been generally indicated by the reference number 1, comprises, as basilar elements thereof, a RF emitter with an associated electronic control device, generally indicated by the reference number 2, a RF source 3, a control CPU 4 and a man/machine interface 5.

Said man/machine interface 5 comprises, for example, a display, an encoder, push-buttons, LED's, indicating elements, and so on.

The control CPU 4 controls the overall apparatus through a built-in firmware/software package and related use programs.

The RF source or generator 3 is controlled by said CPU 4 with a target selection of "transmitters" / "receivers".

Said emitter 2 emits a radiofrequency condition in the radiofrequency range from 500 KHz and 10 MHz, and is designed for generating a temperature increase within the skin tissues.

More specifically, said emitter 2 comprises a plurality of electric contacts 11 made of a biocompatible material, generally indicated by the references RF1, RF2, RF3, and RF4, which connect the radiofrequency source or generator 3 to the human body.

The emitter 2 further comprises an acceleration sensor 6, or other like elements of like function, allowing to detect any emitter displacements.

A temperature sensor 7, either of a pyrometric or an IR type, is herein provided for detecting the temperature increase of the human body part being treated, without physically contacting the human skin.

As shown, the emitter is further coupled to the control CPU 4 through a memory 8, for saving data of the handle 10.

The control apparatus or CPU 4 is based, as stated, on a related operating software/firmware.

Said software, through the CPU and hardware, will be able of detecting if the handle is held at a set position for time longer than that programmed by the apparatus program, that is if it is held at a single position, and will able of locking and/or limiting the RF frequency to prevent any undesired effects from occurring.

The apparatus is moreover adapted to monitor the temperature of the processed area and lock and/or limit the RF emission, to prompt the apparatus operator if said temperature would exceed preset values, and this also to prevent any undesired effects.

That same temperature sensor 7 may also be used by the operator to monitor the temperature increase of the skin region being processed, thereby also monitoring the efficiency of the treatment being performed thereon.

Advantageously, the RF source 2 is coupled to the control apparatus or CPU 4 through electric cables or wires.

In particular, said RF source 2 is coupled to the related generator or source 3 arranged within the apparatus 1, by two high-voltage individually shielded cables to prevent RF emissions from propagating to an encompassing environment and to reduce as far as possible undesired effects on the apparatus operator and on the person being subjected to the treatment.

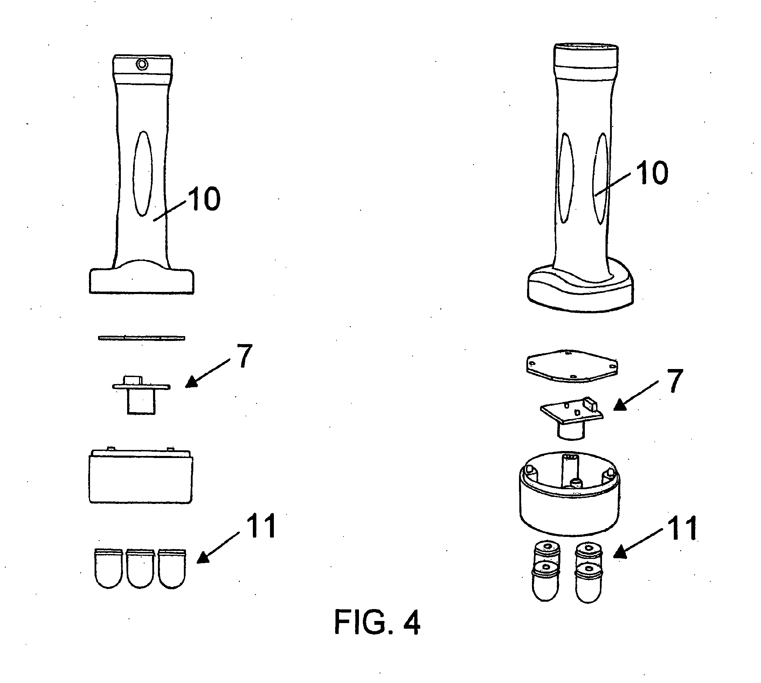

Figure 4 shows the processing handle 10 applied to the inventive apparatus and also shows a diagram of the main elements constituting said handle.

In particular, the handle 10 comprises a movement or displacement sensor 11 and/or a similar electronic/mechanical circuit detecting the movement of the handle 10 and including a store for storing the handle data therein.

The reference number 7 indicates the pyrometric temperature sensor, also adapted to detect or sense the temperature of the skin region being treated.

The reference number 11 shows four biocompatible contacts for transmitting RF power to the human body.

It has been found that the invention fully achieves the intended aim and objects.

In fact, the invention provides a novel RF frequency treating apparatus, in which a set of cooperating electronic technologies and components, such as temperature, movement or displacement sensor, microprocessors and so on are used.

The above components cooperate and interact with one another to reduce to a minimum possible undesired RF side effects, as a RF radiation is applied to a human body to reduce skin blemishes and/or pathologies, while aiding the apparatus operator to perform a truly efficient and safe treatment.

In practicing the invention, the used materials, as well as the size and shapes, can be any, depending on requirements.

## Claims

1. An improved RF apparatus for heat treating slight blemishes and/or pathologies of a human body skin by increasing a concentration of skin existing collagen and generating new collagen formations, said apparatus comprising an apparatus handle including an electronically controlled RF emitter, a RF source, a control device and a man-machine interface, said RF emitter comprising a plurality of electric contacts made of a biocompatible material, said electric contacts coupling said RF source to said human body, a movement sensor to detect a displacement of said emitter, and a temperature sensor to detect a temperature increase of a portion of said human body being treated, **characterised by** said control device being adapted to detect if said handle is held at a set position for a time longer than a preset programmed time to switch off or limit said RF emitter to prevent the skin from being overheated.

2. An improved RF apparatus, according to claim 1, **characterized in that** said man-machine interface comprises a display, an encoder, push-buttons, LED's, and flag or indicating elements.

3. An improved RF apparatus, according to claim 1, **characterized in that** said control device comprises a central processing unit or CPU controlling said apparatus through built-in firmware/software and running programs.

4. An improved RF apparatus, according to claim 3, **characterized in that** said RF source is controlled by said CPU through a transmitter/receiver selection function.

5. An improved RF apparatus, according to one or more of the preceding claims, **characterized in that** said emitter emits a radiofrequency in a frequency range from 500 KHz to 10 MHz, and being designed for increasing the temperature within skin tissues of said human body.

6. An improved RF apparatus, according to one or more of the preceding claims, **characterized in that** said biocompatible material emitter electric contacts connect said RF source to said human body.

7. An improved RF apparatus, according to one or more of the preceding claims, **characterized in that** said emitter movement sensor comprises an acceleration sensor designed for performing a same displacement detecting function as that of said emitter.

8. An improved RF apparatus, according to one or more of the preceding claims, **characterized in that** said temperature sensor is an IR pyrometric sensor for detecting a temperature increase of said human body portion being processed without physically contacting said body portion.

9. An improved RF apparatus, according to one or more of the preceding claims, **characterized in that** said emitter is coupled to said CPU through a coupling memory for saving data of a handle of said apparatus.

10. An improved RF apparatus, according to one or more of the preceding claims, **characterized in that** said RF emitter is coupled to said RF source by two high-voltage individually shielded cables to prevent RF emissions from propagating to an encompassing environment and reduce as far as possible undesired effects on the apparatus operator and on the person being subjected to the treatment.

11. An improved RF apparatus, according to one or more of the preceding claims, **characterized in that** said handle of said apparatus comprises a movement sensor and/or an equivalent electronic circuit and/or mechanical sensor adapted to detect the movement of said handle and further including a memory for saving handle data, said handle further comprising a temperature pyrometric sensor for detecting the temperature of the human body portion being treated and biocompatible material contacts for transmitting RF energy to said human body portion.

## Patentansprüche

1. Verbessertes HF-Gerät zur Wärmebehandlung von leichten Makeln und/oder Pathologien der Haut eines menschlichen Körpers durch Erhöhen der Konzentration an in der Haut vorhandenem Kollagen und Generieren neuer Kollagenbildungen, wobei das Gerät einen Gerätegriff umfasst, der einen elektronisch gesteuerten HF-Emitter, eine HF-Quelle, eine Steuervorrichtung und eine Mensch-Maschine-Schnittstelle umfasst, wobei der HF-Emitter eine Vielzahl von elektrischen Kontakten, die aus einem biokompatiblen Material bestehen, wobei die elektrischen Kontakte die HF-Quelle mit dem menschlichen Körper koppeln, einen Bewegungssensor, um eine Verschiebung des Senders zu ermitteln, und einen Temperatursensor, um einen Temperaturanstieg eines Teils des behandelten menschlichen Körpers zu ermitteln, umfasst, **dadurch gekennzeichnet, dass** die Steuervorrichtung geeignet ist, um zu ermitteln, ob der Griff länger als eine voreingestellte programmierte Zeit lang in einer eingestellten Position gehalten wird, um den HF-Emitter abzuschalten oder einzuschränken, um zu verhindern, dass die Haut überhitzt wird.

2. Verbessertes HF-Gerät nach Anspruch 1, **dadurch gekennzeichnet, dass** die Mensch-Maschine-Schnittstelle ein Display, einen Codierer, Tasten, LEDs und Markierungs- oder Indikatorelemente umfasst.

3. Verbessertes HF-Gerät nach Anspruch 1, **dadurch gekennzeichnet, dass** die Steuervorrichtung eine Zentraleinheit oder CPU umfasst, die das Gerät durch eingebaute Firmware/Software und das Ausführen von Programmen steuert.

4. Verbessertes HF-Gerät nach Anspruch 3, **dadurch gekennzeichnet, dass** die HF-Quelle von der CPU über eine Sender/Empfänger-Auswahlfunktion gesteuert wird.

5. Verbessertes HF-Gerät nach einem oder mehreren der vorhergehenden Ansprüche, **dadurch gekennzeichnet, dass** der Emitter eine Hochfrequenz in einem Frequenzbereich von 500 KHz bis 10 MHz emittiert und ausgelegt ist, um die Temperatur innerhalb von Hautgeweben des menschlichen Körpers zu erhöhen.

6. Verbessertes HF-Gerät nach einem oder mehreren der vorhergehenden Ansprüche, **dadurch gekennzeichnet, dass** die elektrischen Kontakte des Emitters aus biokompatiblem Material die HF-Quelle mit dem menschlichen Körper verbinden.

7. Verbessertes HF-Gerät nach einem oder mehreren der vorhergehenden Ansprüche, **dadurch gekennzeichnet, dass** der Emitterbewegungssensor einen Beschleunigungssensor umfasst, der ausgelegt ist, um dieselbe Verschiebungsermittlungsfunktion wie die des Emitters auszuführen.

8. Verbessertes HF-Gerät nach einem oder mehreren der vorhergehenden Ansprüche, **dadurch gekennzeichnet, dass** der Temperatursensor ein pyrometrischen IR-Sensor zum Ermitteln eines Temperaturanstiegs des behandelten menschlichen Körperteils ohne räumliche Berührung des Körperteils ist.

9. Verbessertes HF-Gerät nach einem oder mehreren der vorhergehenden Ansprüche, **dadurch gekennzeichnet, dass** der Emitter mit der CPU über einen Kopplungsspeicher zum Speichern von Daten eines Griffs des Geräts gekoppelt ist.

10. Verbessertes HF-Gerät nach einem oder mehreren der vorhergehenden Ansprüche, **dadurch gekennzeichnet, dass** der HF-Emitter mit der HF-Quelle durch zwei einzeln abgeschirmte Hochspannungskabel gekoppelt ist, um zu verhindern, dass sich HF-Emissionen auf eine unmittelbare Umgebung ausbreiten, und um unerwünschte Auswirkungen auf den Bediener des Geräts und die Person, die der Behandlung unterzogen wird, möglichst zu reduzieren.

11. Verbessertes HF-Gerät nach einem oder mehreren der vorhergehenden Ansprüche, **dadurch gekennzeichnet, dass** der Griff des Geräts einen Bewegungssensor und/oder eine elektronische Ersatzschaltung und/oder einen mechanischen Sensor aufweist, der bzw. die geeignet ist bzw. sind, um die Bewegung des Griffs zu ermitteln, und ferner einen Speicher umfasst, um Griffdaten zu speichern, wobei der Griff ferner einen pyrometrischen Temperatursensor zum Ermitteln der Temperatur des behandelten menschlichen Körperteils und Kontakte aus biokompatiblem Material zum Übertragen von HF-Energie auf den menschlichen Körperteil umfasst.

## Revendications

1. Appareil RF amélioré pour le traitement thermique d'imperfections légères et/ou de pathologies de la peau du corps humain, en augmentant une concentration de collagène existant dans la peau et en générant de nouvelles productions de collagène, ledit appareil comprenant une poignée, incluant un émetteur RF commandé électroniquement, une source RF, un dispositif de commande et une interface homme-machine, ledit émetteur RF comprenant une pluralité de contacts électriques réalisés en un matériau biocompatible, lesdits contacts électriques couplant ladite source RF audit corps humain, un détecteur de mouvement pour détecter un déplacement dudit émetteur, et un capteur de température pour détecter une augmentation de température d'une partie dudit corps humain traité, **caractérisé en ce que** ledit dispositif de commande est conçu pour détecter si ladite poignée est maintenue à une position définie pendant un temps plus long qu'un temps programmé préétabli, afin d'arrêter ou de limiter ledit émetteur RF pour empêcher la peau d'être trop chauffée.

2. Appareil RF amélioré, selon la revendication 1, **caractérisé en ce que** ladite interface homme-machine comprend un écran, un codeur, des boutons poussoirs, des DEL, et un drapeau ou des éléments indicateurs.

3. Appareil RF amélioré, selon la revendication 1, **caractérisé en ce que** ledit dispositif de commande comprend une unité centrale de traitement ou CPU commandant ledit appareil par des microprogrammes/logiciels intégrés et des programmes d'exploitation.

4. Appareil RF amélioré, selon la revendication 3, **caractérisé en ce que** ladite source RF est commandée par ledit CPU par une fonction de sélection émetteur/récepteur.

5. Appareil RF amélioré, selon une ou plusieurs des revendications précédentes, **caractérisé en ce que** ledit émetteur émet une radiofréquence dans une plage de fréquences comprise entre 500 KHz et 10 MHz, et **en ce qu'**il est conçu pour augmenter la température des tissus de la peau dudit corps humain.

6. Appareil RF amélioré, selon une ou plusieurs des revendications précédentes, **caractérisé en ce que** lesdits contacts électriques de l'émetteur, en un matériau biocompatible, relient ladite source RF audit corps humain.

7. Appareil RF amélioré, selon une ou plusieurs des revendications précédentes, **caractérisé en ce que** ledit détecteur de mouvement de l'émetteur comprend un capteur d'accélération conçu pour réaliser une même fonction de détection de déplacement que celle dudit émetteur.

8. Appareil RF amélioré, selon une ou plusieurs des revendications précédentes, **caractérisé en ce que** ledit capteur de température est un capteur pyrométrique IR pour détecter une augmentation de température de ladite partie du corps humain traitée sans contact physique de ladite partie du corps.

9. Appareil RF amélioré, selon une ou plusieurs des revendications précédentes, **caractérisé en ce que** ledit émetteur est couplé audit CPU par une mémoire de couplage pour enregistrer les données d'une poignée dudit appareil.

10. Appareil RF amélioré, selon une ou plusieurs des revendications précédentes, **caractérisé en ce que** ledit émetteur RF est couplé à ladite source RF par deux câbles haute tension blindés séparément pour éviter que les émissions RF ne se propagent dans l'environnement et réduire dans la mesure du possible les effets indésirables sur l'opérateur de l'appareil et sur la personne soumise au traitement.

11. Appareil RF amélioré, selon une ou plusieurs des revendications précédentes, **caractérisé en ce que** ladite poignée dudit appareil comprend un détecteur de mouvement et/ou un circuit électronique équivalent et/ou un capteur mécanique conçu pour détecter le mouvement de ladite poignée et comprenant, en outre, une mémoire pour enregistrer les données de la poignée, ladite poignée comprenant, en outre, un capteur pyrométrique de température pour détecter la température de la partie du corps humain traitée et des contacts en matériau biocompatible pour transmettre l'énergie RF à ladite partie du corps humain.
